(19)

Europäisches
Patentamt
European
Patent Office
Office européen
des brevets

(11) **EP 4 534 979 A1**

(12) **EUROPEAN PATENT APPLICATION**
published in accordance with Art. 153(4) EPC

(43) Date of publication:
**09.04.2025 Bulletin 2025/15**

(21) Application number: **23864451.2**

(22) Date of filing: **30.06.2023**

(51) International Patent Classification (IPC):
*G01N 21/31* (2006.01)

(52) Cooperative Patent Classification (CPC):
**G01N 21/3151; G01N 21/274; G01N 33/49;**
G01N 2015/055; G01N 2201/0662

(86) International application number:
**PCT/CN2023/105029**

(87) International publication number:
**WO 2024/055714 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.09.2022 CN 202211122612**

(71) Applicant: **Shenzhen Drawray Biotech Co., Ltd.
Shenzhen, Guangdong 518116 (CN)**

(72) Inventors:
• **LIU, Yudong
Shenzhen, Guangdong 518116 (CN)**
• **WANG, Guangliang
Shenzhen, Guangdong 518116 (CN)**

(74) Representative: **Mewburn Ellis LLP
Aurora Building
Counterslip
Bristol BS1 6BX (GB)**

(54) **WHOLE BLOOD HEMATOCRIT MEASUREMENT METHOD**

(57) A whole blood hematocrit measurement method and apparatus. The method comprises: injecting a first liquid to be subjected to measurement into a measuring container (200); measuring, in a first direction and a second direction, light intensity data D11 and light intensity data D12 of said first liquid; cleaning the measuring container (200), and injecting a second liquid to be subjected to measurement into the measuring container (200); measuring, in the first direction and the second direction, light intensity data D21 and light intensity data D22 of said second liquid; and calculating absorbance A by means of D11, D12, D21 and D22.

```
┌────────────────────────────────┐
│ Injecting a first to-be-measured│
│ liquid into a measuring          │
│ container                        │
└────────────────────────────────┘
              │
              ▼
┌────────────────────────────────┐
│ Measuring light intensity data   │
│ D11 and light intensity data D12 │
│ of the first to-be-measured      │
│ liquid from a first direction    │
│ and a second direction,          │
│ respectively                     │
└────────────────────────────────┘
              │
              ▼
┌────────────────────────────────┐
│ Cleaning the measuring container,│
│ and injecting a second           │
│ to-be-measured liquid into the   │
│ measuring container              │
└────────────────────────────────┘
              │
              ▼
┌────────────────────────────────┐
│ Measuring light intensity data   │
│ D21 and light intensity data D22 │
│ of the second to-be-measured     │
│ liquid from the first direction  │
│ and the second direction,        │
│ respectively                     │
└────────────────────────────────┘
              │
              ▼
┌────────────────────────────────┐
│ Calculating an absorbance A based│
│ on the light intensity data D11, │
│ the light intensity data D12, the│
│ light intensity data D21, and the│
│ light intensity data D22         │
└────────────────────────────────┘
              │
              ▼
┌────────────────────────────────┐
│ Obtaining hematocrit from a      │
│ relationship curve according to  │
│ the absorbance A                 │
└────────────────────────────────┘
```

FIG. 1

EP 4 534 979 A1

## Description

The present application claims the priority of Chinese patent application No. 2022111226129, filed with SIPO of China on September 15, 2022, and entitled "METHOD FOR MEASURING WHOLE BLOOD HEMATOCRIT", which is incorporated herein by reference in its entirety.

## TECHNICAL FIELD

The invention relates to the technical field of medical detection, and in particular to a method for measuring a whole blood hematocrit.

## BACKGROUND

An HCT, namely a hematocrit, refers to a proportion of red blood cells in a whole blood sample of a certain volume. Traditional chemiluminescence instruments measure the HCT in whole blood samples in other manners outside the instruments, and the Wintobe-landsbrey method is usually used to measure the HCT. However, the Wintobe-landsbrey method is complicated to operate, and it takes a long time to measure. In addition, measured results need to be manually input into the chemiluminescence instrument to correct the measured results of the item, thus the measurement operation is complicated and inefficient. Some chemiluminescence instrument manufacturers integrate a hematocrit module in each instrument to detect the hematocrit of whole blood samples by impedance measurement. In the manner of impedance measurement, the presence of many small gaps in a measuring container increases the difficulty of cleaning the measuring container during repeated measurements. Furthermore, the detecting electrode needs to be in contact with the whole blood sample, thus a long-term usage can easily cause corrosion and aging of the detecting electrode.

## SUMMARY

In view of this, an embodiment of the present application provides a method for measuring whole blood hematocrit and a device for measuring whole blood hematocrit, so as to address the following problems in the traditional method for measuring hematocrit: the operation performed in manner of measuring outside the instrument is complicated, and it takes a long time to measure, and the measured results need to be manually input into a chemiluminescence instrument to correct the measured results, and thus the efficiency is low; and in an impedance measurement manner, the presence of many small gaps in a measuring container increases the difficulty of cleaning the measuring container during repeated measurements, and a detecting electrode needs to be in contact with a whole blood sample, thus a long-term usage can easily cause corrosion and aging of the detecting electrode. In the method for measuring whole blood hematocrit of an embodiment of the present application, a measuring container having no small gaps is used and thus can reduce the difficulty of cleaning the measuring container, corrosion and aging of the detector can be avoided, and a measurement for the hematocrit in the whole blood sample can be realized. Furthermore, in an embodiment of the present application, dual wavelengths are used for measurement, thereby effectively eliminating an influence of interferences in the sample and improving accuracy of the measurement.

A method for measuring whole blood hematocrit, including steps of:

injecting a first to-be-measured liquid into a measuring container; measuring light intensity data D11 and light intensity data D12 of the first to-be-measured liquid from a first direction and a second direction, respectively;
cleaning the measuring container, and injecting a second to-be-measured liquid into the measuring container; measuring light intensity data D21 and light intensity data D22 of the second to-be-measured liquid from the first direction and the second direction, respectively;
calculating an absorbance A based on the light intensity data D11, the light intensity data D12, the light intensity data D21, and the light intensity data D22, where a formula for calculating the absorbance A is:

$$A = K_1 \times (\lg \frac{D_{11} - D_{Dark}}{D_{12} - D_{Dark}} - \lg \frac{D_{21} - D_{Dark}}{D_{22} - D_{Dark}})$$

where $K_1$ denotes a conversion factor of the measuring container (200), and $D_{Dark}$ denotes a dark current reading on a detecting system; and
obtaining hematocrit from a relationship curve according to the absorbance A.

In some embodiments, before injecting the first to-be-measured liquid into the measuring container, the method further includes a step of: injecting a cleaning solution into the measuring container to clean the measuring container.

In some embodiments, the first direction and the second direction are two directions perpendicular to each other on a horizontal plane.

In some embodiments, making the relationship curve includes steps of:

measuring absorbances corresponding to different concentrations by using a standard solution, and drawing the relationship curve based on a relationship between concentration and absorbance; where a formula of the relationship curve is:

$$HCT = k_2 \times e^{b \times A}$$

Where, $k_2$ and $b$ are fitting parameters obtained by exponential curve fitting after obtaining a calibration curve of absorbance and hematocrit by measuring absorbances of hematocrit samples of 10%, 20%, 40%, 60% and 80%, respectively.

In some embodiments, the measuring container is a cuvette.

In some embodiments, an optical path length of the measuring container is 4 mm, and $K_1 = 2.5$.

In some embodiments, injecting the first to-be-measured liquid into the measuring container and injecting the second to-be-measured liquid into the measuring container are performed by a liquid injection mechanism.

An embodiment of the present application further provides a device for measuring whole blood hematocrit. The measuring container of the device for measuring whole blood hematocrit has no small gaps, which can reduce the difficulty of cleaning the measuring container, avoid the corrosion and aging of the detector, and realize the hematocrit measurement of the whole blood sample. Furthermore, the device for measuring whole blood hematocrit of the present invention performs measurement by using dual wavelengths, which can effectively eliminate the influence of interferences in the samples and improve the accuracy of the measurement.

The device for measuring whole blood hematocrit of an embodiment of the present application includes a first signal generating end, a first signal receiving end, a second signal generating end, a second signal receiving end and a detector. The first signal generating end is arranged opposite to the first signal receiving end, and the second signal generating end is arranged opposite to the second signal receiving end. The detector is electrically connected to the first signal generating end, the first signal receiving end, the second signal generating end and the second signal receiving end. The first signal generating end and the first signal receiving end are configured to measure light intensity data D11 of a first to-be-measured liquid and light intensity data D21 of a second to-be-measured liquid from a first direction in a horizontal plane, respectively, and the second signal generating end and the second signal receiving end are configured to measure light intensity data D12 of the first to-be-measured liquid and light intensity data D22 of the second to-be-measured liquid from a second direction in the horizontal plane, respectively.

In some embodiments, the first direction and the second direction are two directions perpendicular to each other on the horizontal plane.

In some embodiments, the device for measuring whole blood hematocrit further includes a measuring container.

In some embodiments, the device for measuring whole blood hematocrit further includes a liquid injection mechanism, and the liquid injection mechanism is configured to inject liquid into the measuring container.

In the above-mentioned method for measuring whole blood hematocrit, the measuring container has no small gaps, which can reduce the difficulty of cleaning the measuring container, avoid the corrosion and aging of the detector, and realize the hematocrit measurement of the whole blood sample. Furthermore, the dual wavelengths of 420nm and 540nm are used for measurement in the present invention, which can effectively eliminate the influence of interferences in the samples and improve the accuracy of the measurement.

BRIEF DESCRIPTION OF THE DRAWINGS

In order to illustrate the technical solutions in the embodiments of the present application more clearly, the drawings need to be used in the description of the embodiments will be briefly described hereinafter. Obviously, the drawings described hereinafter are merely embodiments of the present application, and for those ordinary skilled in the art, other drawings may be obtained based on the drawings without any creative effects.

In order to make the present application and beneficial effects thereof to be more completely understood, the following description will be illustrated in conjunction with the accompanying drawings. In the following description, the same reference numerals represent the same objects.

FIG. 1 is a schematic flow chart of a method for measuring whole blood hematocrit according to some embodiments of the present application;

FIG. 2 is a schematic view showing a device for measuring whole blood hematocrit according to some embodiments of the present application;

FIG. 3 is a schematic view showing part of the device for measuring whole blood hematocrit according to some embodiments of the present application.

Description of Reference Numerals

101, first signal generating end; 102, first signal receiving end; 103, second signal generating end; 104, second signal receiving end; 200, measuring container.

DETAILED DESCRIPTION OF THE EMBODIMENTS

In order to make the above-mentioned objectives, features and advantages of the present invention more obvious and to be better understood, the specific embodiments of the present invention are described in detail below in conjunction with the accompanying drawings. In the following description, many specific details are set forth to facilitate a full understanding of the present invention. However, the present invention can be implemented in many other ways different from those described herein, and those skilled in the art can make similar improvements without violating the connotation of the present invention, so the present invention is not limited by the specific embodiments disclosed hereinafter.

In the description of the present invention, it should be understood that the terms "center", "longitudinal", "lateral", "length", "width", "thickness", "up", "down", "front", "back", "left", "right", "vertical", "horizontal", "top", "bottom", "inside", "outside", "clockwise", "counterclockwise", "axial", "radial", "circumferential" and the like indicate orientations or positional relationships based on the orientations or positional relationships shown in the accompanying drawings are used only for the convenience of describing the present invention and simplifying the description, but do not indicate or imply that a referred device or element must have a specific orientation, be constructed and operated in a specific orientation, and therefore should not be understood as limiting the present invention.

In addition, the terms "first" and "second" are used for descriptive purposes only and should not be understood as indicating or implying relative importance or implicitly indicating the number of the indicated technical features. Therefore, the features defined as "first" and "second" may explicitly or implicitly include at least one of the features. In the description of the present invention, "plurality" means at least two, such as two, three, etc., unless otherwise clearly and specifically defined.

In the present invention, unless otherwise clearly specified and limited, the terms "install", "connect", "attach", "fix" and the like should be understood in a broad sense, for example, it can be a fixed connection, a detachable connection, or an integral connection; it can be a mechanical connection or an electrical connection; it can be a direct connection, or an indirect connection through an intermediate medium, or it can be an internal communication of two elements or an interaction relationship between two elements, unless otherwise clearly defined. For the ordinary skilled in the art, the specific meanings of the above terms in the present invention can be understood according to specific circumstances.

In the present invention, unless otherwise clearly specified and limited, a first feature being "on" or "below" a second feature can mean that the first and second features are in direct contact, or the first and second features are in indirect contact through an intermediate medium. Moreover, a first feature being "above", "over" or "on" the second feature may mean that the first feature is directly above or obliquely above the second feature, or simply means that a horizontal height of the first feature is higher than that of the second feature. A first feature being "below", "under" or "beneath" a second feature may mean that the first feature is directly below or obliquely below the second feature, or simply means that the horizontal height of the first feature is less than that of the second feature.

It should be noted that when an element is referred to as being "fixed to" or "disposed on" another element, it may be directly on the other element or there may be an intermediate element. When an element is considered to be "connected to" another element, it may be directly connected to the other element or there may be an intermediate element. The terms "vertical", "horizontal", "up", "down", "left", "right" and similar expressions used herein are for illustrative purposes only but are not intended to be a unique implementation method.

In the description of the present invention, " plurality " means more than one, "several" means more than two, "greater than", "less than", "exceed", etc. are understood to exclude the number itself, and "above", "below", "within", etc. are understood to include the number itself. If there is a description of "first" or "second", it is only used for the purpose of distinguishing the technical features, but cannot be understood as indicating or implying the relative importance or implicitly indicating the number of the indicated technical features or implicitly indicating the order of the indicated technical features.

Unless otherwise defined, all technical and scientific terms used herein have the same meaning as those commonly understood by those skilled in the art to which the present invention belongs. The terms used herein in the description of the present invention are only for the purpose of describing specific embodiments but are not intended to limit the present invention. The term "and/or" used herein includes any and all combinations of one or more of the related listed objects.

Some embodiments of the present application provide a method for measuring whole blood hematocrit to solve the following problems in the traditional method for measuring hematocrit: the operation performed in the manner of measuring outside the instrument is complicated, and it takes a long time to measure, and the measured results need to be manually input into the chemiluminescence instrument to correct the measured results, and thus the efficiency is low; and in an impedance measurement manner, the presence of many small gaps in the measuring container 200 increases the difficulty of cleaning the measuring container 200 during repeated measurements, and the detecting electrode needs to be in contact with the whole blood sample, thus the long-term usage can easily cause the corrosion and aging of the detecting electrode. The description will be made in conjunction with the accompanying drawings.

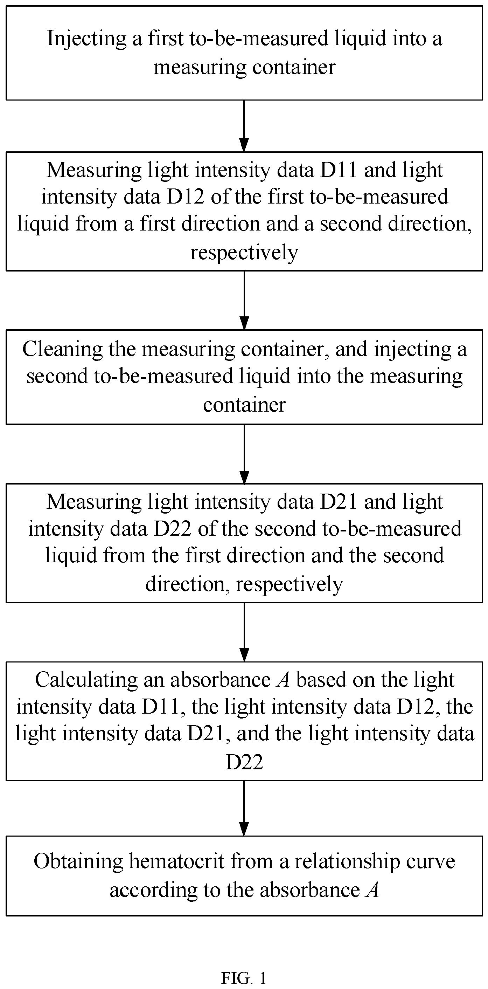

In some embodiments of the present application, the method for measuring whole blood hematocrit is provided. Illustratively, referring to FIG. 1, which is a schematic flow chart of a method for measuring whole blood hematocrit according to some embodiments of the present application, the method for measuring whole blood hematocrit of the present application can be used for measuring the whole blood hematocrit.

In order to more clearly illustrate a process of the method for measuring whole blood hematocrit, the method for measuring whole blood hematocrit will be introduced below with reference to the accompanying drawings.

Illustratively, a method for measuring whole blood hematocrit includes the following steps.

In Step 1, inject a first to-be-measured liquid into the measuring container 200, and measure light intensity data D11 and light intensity data D12 of the first to-be-measured liquid from a first direction and a second direction respectively.

In Step 2, clean the measuring container 200, inject a second to-be-measured liquid into the measuring container 200, and measure light intensity data D21 and light intensity data D22 of the second to-be-measured liquid from the first direction and the second direction respectively.

In Step 3, calculate an absorbance A based on the light intensity data D11, the light intensity data D12, the light intensity data D21, and the light intensity data D22. The formula for calculating the absorbance *A* is:

$$A = K_1 \times (\lg \frac{D_{11} - D_{Dark}}{D_{12} - D_{Dark}} - \lg \frac{D_{21} - D_{Dark}}{D_{22} - D_{Dark}})$$

Where $K_1$ denotes a conversion factor of the measuring container 200, and $D_{Dark}$ denotes a dark current reading on a detecting system.

In Step 4, obtain the hematocrit from a relationship curve according to the absorbance *A*.

In some embodiments, before injecting the first to-be-measured liquid into the measuring container 200, the following step is further included: injecting a cleaning solution into the measuring container 200 to clean the measuring container 200.

In some embodiments, the first direction and the second direction are two directions perpendicular to each other on a horizontal plane.

In some embodiments, making the relationship curve includes the following step:

The absorbances corresponding to different concentrations are measured by using a standard solution, and the relationship curve is obtained based on a relationship between concentration and absorbance. The formula of the relationship curve is:

$$HCT = k_2 \times e^{b \times A}$$

where, $k_2$ and $b$ are fitting parameters obtained by exponential curve fitting after obtaining a calibration curve of absorbance and hematocrit by measuring the absorbances of the hematocrit samples of 10%, 20%, 40%, 60% and 80% respectively.

In some embodiments, the measuring container 200 is a cuvette.

In some embodiments, an optical path length of the measuring container 200 is equal to 4 mm, and $K_1$ = 2.5.

In some embodiments, injecting the first to-be-measured liquid into the measuring container 200 and injecting the second to-be-measured liquid into the measuring container 200 are performed by a liquid injection mechanism.

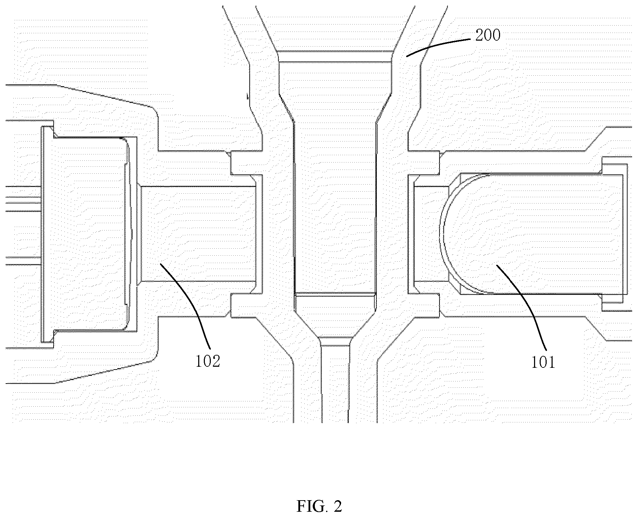

In some of the embodiments, illustratively, please refer to FIG. 1 and FIG. 2, which are schematic views showing structures of a device for measuring whole blood hematocrit used in the method for measuring whole blood hematocrit according to the embodiments of the present application.

In some embodiments, the device for measuring whole blood hematocrit used in the method for measuring whole blood hematocrit includes a first signal generating end 101, a first signal receiving end 102, a second signal generating end 103, a second signal receiving end 104 and a detector. The first signal generating end 101 is arranged opposite to the first signal receiving end 102, and the second signal generating end 103 is arranged opposite to the second signal receiving end 104. The area enclosed by the first signal generating end 101, the first signal receiving end 102, the second signal generating end 103 and the second signal receiving end 104 is a detection station. The detector is electrically connected to the first signal generating end 101, the first signal receiving end 102, the second signal generating end 103 and the second signal receiving end 104.

In some embodiments, the device for measuring whole blood hematocrit further includes a measuring container 200.

In some of the embodiments, the device for measuring whole blood hematocrit further includes a liquid injection mechanism, which is configured to inject liquid into the measuring container 200.

Embodiment 1

This embodiment provides a method for measuring whole blood hematocrit.

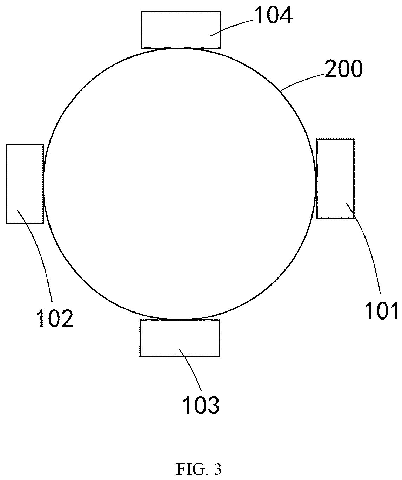

The method for measuring whole blood hematocrit of this embodiment uses the device for measuring whole blood hematocrit shown in FIGS. 2 and 3. The device for measuring whole blood hematocrit includes the first signal generating end 101, the first signal receiving end 102, the second signal generating end 103, the second signal receiving end 104, the measuring container 200, the liquid injection mechanism and the detector. The first signal generating end 101 is arranged opposite to the first signal receiving end 102, and the second signal generating end 103 is arranged opposite to the second signal receiving end 104. The area enclosed by the first signal generating end 101, the first signal receiving end 102, the second signal generating end 103, and the second signal receiving end 104 is the detection station. The detector is electrically connected to the first signal generating end 101, the first signal receiving end 102, the second signal generating end 103 and the second signal receiving end 104. The liquid injection mechanism is configured to inject liquid into the measuring container 200.

The method for measuring whole blood hematocrit includes the following steps.

In Step 1, inject a cleaning solution into the measuring container 200 through the liquid injection mechanism to clean the measuring container 200.

In Step 2, inject the first to-be-measured liquid into the measuring container 200 through the liquid injection mechanism, and measure the light intensity data D11 and the light intensity data D12 of the first to-be-measured liquid from the first direction and the second direction perpendicular to each other on the horizontal plane, respectively.

In Step 3, clean the measuring container 200, and inject the second to-be-measured liquid into the measuring container 200 through the liquid injection mechanism, and measure the light intensity data D21 and the light intensity data D22 of the second to-be-measured liquid from the first direction and the second direction perpendicular to each other on the horizontal plane, respectively.

In Step 4, calculate the absorbance $A$ based on the light intensity data D11, the light intensity data D12, the light intensity data D21, and the light intensity data D22. The formula for calculating the absorbance $A$ is:

$$A = K_1 \times (\lg \frac{D_{11} - D_{Dark}}{D_{12} - D_{Dark}} - \lg \frac{D_{21} - D_{Dark}}{D_{22} - D_{Dark}})$$

Wherein, $K_1$ denotes the conversion factor of the measuring container 200. The optical path length of the measuring container 200 is equal to 4 mm, and $K_1 = 2.5$. $D_{Dark}$ denotes the dark current reading on the detecting system.

In Step 5, measure the absorbances corresponding to different concentrations by using the standard solution, and draw the relationship curve based on the relationship between concentration and absorbance, The formula of the relationship curve is:

$$HCT = k_2 \times e^{b \times A}$$

where, $k_2$ and $b$ are the fitting parameters obtained by exponential curve fitting after obtaining the calibration curve of absorbance and hematocrit by measuring the absorbances of the hematocrit samples of 10%, 20%, 40%, 60% and 80% respectively.

In Step 6, obtain the hematocrit from the relationship curve according to the absorbance $A$.

Embodiment 2

This embodiment provides a method for measuring whole blood hematocrit.

The method for measuring whole blood hematocrit of this embodiment uses the device for measuring whole blood hematocrit shown in FIGS. 2 and 3. The device for measuring whole blood hematocrit includes the first signal generating end 101, the first signal receiving end 102, the second signal generating end 103, the second signal receiving end 104, the measuring container 200, the liquid injection mechanism and the detector. The first signal generating end 101 is arranged opposite to the first signal receiving end 102, and the second signal generating end 103 is arranged opposite to the second signal receiving end 104. The area enclosed by the first signal generating end 101, the first signal receiving end 102, the second signal generating end 103, and the second signal receiving end 104 is the detection station. The detector is electrically connected to the first signal generating end 101, the first signal receiving end 102, the second signal generating end 103 and the second signal receiving end 104. The liquid injection mechanism is configured to inject liquid into the measuring container 200.

The method for measuring whole blood hematocrit includes the following steps.

In Step 1, inject a cleaning solution into the measuring container 200 through the liquid injection mechanism to clean the measuring container 200.

In Step 2, inject the first to-be-measured liquid into the measuring container 200 through the liquid injection mechanism, and measure the light intensity data D11 and the light intensity data D12 of the first to-be-measured liquid from the first direction and the second direction perpendicular to each other on the horizontal plane, respectively.

In Step 3, clean the measuring container 200, and inject the second to-be-measured liquid into the measuring container 200 through the liquid injection mechanism, and measure the light intensity data D21 and the light intensity data D22 of the second to-be-measured liquid from the first direction and the second direction perpendicular to each other on the horizontal plane, respectively.

In Step 4, calculate the absorbance A based on the light intensity data D11, the light intensity data D12, the light intensity data D21, and the light intensity data D22. The formula for calculating the absorbance A is:

$$A = K_1 \times (\lg \frac{D_{11} - D_{Dark}}{D_{12} - D_{Dark}} - \lg \frac{D_{21} - D_{Dark}}{D_{22} - D_{Dark}})$$

Wherein, $K_1$ denotes the conversion factor of the measuring container 200. The optical path length of the measuring container 200 is equal to 4 mm, and $K_1 = 2.5$. $D_{Dark}$ denotes the dark current reading on the detecting system.

In Step 5, measure the absorbances corresponding to different concentrations by using the standard solution, and draw the relationship curve based on the relationship between concentration and absorbance, The formula of the relationship curve is:

$$HCT = k_2 \times e^{b \times A}$$

where, $k_2$ and $b$ are the fitting parameters obtained by exponential curve fitting after obtaining the calibration curve of absorbance and hematocrit by measuring the absorbances of the hematocrit samples of 10%, 20%, 40%, 60% and 80% respectively.

In Step 6, obtain the hematocrit from the relationship curve according to the absorbance A.

After measuring 20 whole blood samples through this embodiment, the measured data are shown in Tables 1 and 2.

Table 1

| | Sample 1 | Sample 2 | Sample 3 | Sample 4 | Sample 5 | Sample 6 | Sample 7 | Sample 8 | Sample 9 | Sample 10 |
|---|---|---|---|---|---|---|---|---|---|---|
| Accurate Hematocrit | 38.0% | 46.0% | 41.0% | 44.0% | 34.5% | 42.5% | 44.0% | 50.0% | 44.0% | 44.0% |
| First Measurement | 37.20% | 41.20% | 44.10% | 47.80% | 35.60% | 38.70% | 41.50% | 50.70% | 46.50% | 42.90% |
| Second Measurement | 39.60% | 43.10% | 40.90% | 46.80% | 33.80% | 39.60% | 41.10% | 51.70% | 47.60% | 44.00% |
| Third Measurement | 35.90% | 44.60% | 44.50% | 48.30% | 33.10% | 44.00% | 40.60% | 51.20% | 44.40% | 41.90% |
| Average Value | 37.57% | 42.97% | 43.17% | 47.63% | 34.17% | 40.77% | 41.07% | 51.20% | 46.17% | 42.93% |
| Standard Deviation | 1.88% | 1.70% | 1.97% | 0.76% | 1.29% | 2.84% | 0.45% | 0.50% | 1.63% | 1.05% |
| Coefficient of Variation | 5.00% | 3.97% | 4.57% | 1.60% | 3.77% | 6.96% | 1.10% | 0.98% | 3.52% | 2.45% |
| Relative Deviation | -1.14% | -6.59% | 5.28% | 8.26% | -0.97% | -4.08% | -6.67% | 2.40% | 4.92% | -2.42% |

Table 2

| | Sample 11 | Sample 12 | Sample 13 | Sample 14 | Sample 15 | Sample 16 | Sample 17 | Sample 18 | Sample 19 | Sample 20 |
|---|---|---|---|---|---|---|---|---|---|---|
| Accurate Hematocrit | 41.0% | 45.0% | 42.0% | 45.5% | 24.0% | 46.5% | 51.5% | 44.5% | 44.0% | 34.0% |
| First Measurement | 44.80% | 43.00% | 42.70% | 45.50% | 23.56% | 45.20% | 58.30% | 44.90% | 44.80% | 34.80% |
| Second Measurement | 45.20% | 46.80% | 39.40% | 43.90% | 24.90% | 44.90% | 55.30% | 38.40% | 45.00% | 34.50% |
| Third Measurement | 43.80% | 42.10% | 37.80% | 41.90% | 25.60% | 46.10% | 56.10% | 40.00% | 42.80% | 36.40% |
| Average Value | 44.60% | 43.97% | 39.97% | 43.77% | 24.69% | 45.40% | 56.57% | 41.10% | 44.20% | 35.23% |
| Standard Deviation | 0.72% | 2.49% | 2.50% | 1.80% | 1.04% | 0.62% | 1.55% | 3.39% | 1.22% | 1.02% |
| Coefficient of Variation | 1.62% | 5.67% | 6.25% | 4.12% | 4.20% | 1.38% | 2.75% | 8.24% | 2.75% | 2.90% |
| Relative Deviation | 8.78% | -2.30% | -4.84% | -3.81% | 2.86% | -2.37% | 9.84% | -7.64% | 0.45% | 3.63% |

It can be seen from the data in Table 1 and Table 2 that in this embodiment, twenty whole blood samples were measured, each sample was measured for three times, and all data obtained for three times were valid.

In summary, in the above-mentioned method for measuring whole blood hematocrit, the measuring container 200 used in the method for measuring whole blood hematocrit of the present invention has no small gaps, which can reduce the difficulty of cleaning the measuring container 200, avoid the corrosion and aging of the detector, and realize the hematocrit measurement of the whole blood sample. Furthermore, dual wavelengths of 420nm and 540nm are used for measurement in the present invention, which can effectively eliminate the influence of interferences in the samples and improve the accuracy of the measurement.

In the above embodiments, the description of each embodiment has its own emphasis. For parts that are not described in detail in one embodiment, reference can be made to the relevant descriptions of other embodiments.

The technical features of the above-described embodiments may be arbitrarily combined. To make the description concise, not all possible combinations of the technical features of the embodiments above are described. However, as long as there is no contradiction in the combinations of these technical features, these combinations should be considered to be within the scope of this specification.

The above-mentioned embodiments are only several implementation methods of the present invention, and the description thereof is relatively specific and detailed, but it cannot be understood as limiting the patent scope of the present invention. It should be noted that, for the ordinary skilled in the art, several variations and improvements can be made without departing from the concept of the present invention, and these variations and improvements all belong to the protection scope of the present invention. Therefore, the patent protection scope of the present invention shall be subject to the attached claims.

**Claims**

1. A method for measuring whole blood hematocrit, **characterized by** comprising steps of:

    injecting a first to-be-measured liquid into a measuring container;
    measuring light intensity data D11 and light intensity data D12 of the first to-be-measured liquid from a first direction and a second direction, respectively;
    cleaning the measuring container, and injecting a second to-be-measured liquid into the measuring container;
    measuring light intensity data D21 and light intensity data D22 of the second to-be-measured liquid from the first direction and the second direction, respectively;
    calculating an absorbance A based on the light intensity data D11, the light intensity data D12, the light intensity data D21, and the light intensity data D22, wherein a formula for calculating the absorbance $A$ is:

$$A = K_1 \times (\lg \frac{D_{11} - D_{Dark}}{D_{12} - D_{Dark}} - \lg \frac{D_{21} - D_{Dark}}{D_{22} - D_{Dark}})$$

    wherein $K_1$ denotes a conversion factor of the measuring container, and $D_{Dark}$ denotes a dark current reading on a detecting system; and
    obtaining hematocrit from a relationship curve according to the absorbance $A$.

2. The method for measuring whole blood hematocrit according to claim 1, wherein before injecting the first to-be-measured liquid into the measuring container, the method further comprises a step of: injecting a cleaning solution into the measuring container to clean the measuring container.

3. The method for measuring whole blood hematocrit according to claim 1, wherein the first direction and the second direction are two directions perpendicular to each other on a horizontal plane.

4. The method for measuring whole blood hematocrit according to any one of claims 1 to 3, wherein making the relationship curve comprises steps of:

    measuring absorbances corresponding to different concentrations by using a standard solution, and drawing the relationship curve based on a relationship between concentration and absorbance;
    wherein a formula of the relationship curve is:

$$HCT = k_2 \times e^{b \times A}$$

wherein, $k_2$ and $b$ are fitting parameters obtained by exponential curve fitting after obtaining a calibration curve of absorbance and hematocrit by measuring absorbances of hematocrit samples of 10%, 20%, 40%, 60% and 80%, respectively.

5. The method for measuring whole blood hematocrit according to any one of claims 1 to 3, wherein the measuring container is a cuvette.

6. The method for measuring whole blood hematocrit according to any one of claims 1 to 3, wherein an optical path length of the measuring container is 4 mm, and $K_1$ = 2.5.

7. The method for measuring whole blood hematocrit according to any one of claims 1 to 3, wherein injecting the first to-be-measured liquid into the measuring container and injecting the second to-be-measured liquid into the measuring container are performed by a liquid injection mechanism.

8. A device for measuring whole blood hematocrit, **characterized by** comprising a first signal generating end, a first signal receiving end, a second signal generating end, a second signal receiving end, and a detector; the first signal generating end is arranged opposite to the first signal receiving end, and the second signal generating end is arranged opposite to the second signal receiving end; the detector is electrically connected to the first signal generating end, the first signal receiving end, the second signal generating end and the second signal receiving end; the first signal generating end and the first signal receiving end are configured to measure light intensity data D11 of a first to-be-measured liquid and light intensity data D21 of a second to-be-measured liquid from a first direction in a horizontal plane, respectively; the second signal generating end and the second signal receiving end are configured to measure light intensity data D12 of the first to-be-measured liquid and light intensity data D22 of the second to-be-measured liquid from a second direction in the horizontal plane, respectively.

9. The device for measuring whole blood hematocrit according to claim 8, wherein the first direction and the second direction are two directions perpendicular to each other on the horizontal plane.

10. The device for measuring whole blood hematocrit according to claim 8 or 9, wherein the device for measuring whole blood hematocrit further comprises a measuring container.

11. The device for measuring whole blood hematocrit according to claim 10, wherein the device for measuring whole blood hematocrit further comprises a liquid injection mechanism, and the liquid injection mechanism is configured to inject liquid into the measuring container.

Injecting a first to-be-measured liquid into a measuring container

↓

Measuring light intensity data D11 and light intensity data D12 of the first to-be-measured liquid from a first direction and a second direction, respectively

↓

Cleaning the measuring container, and injecting a second to-be-measured liquid into the measuring container

↓

Measuring light intensity data D21 and light intensity data D22 of the second to-be-measured liquid from the first direction and the second direction, respectively

↓

Calculating an absorbance $A$ based on the light intensity data D11, the light intensity data D12, the light intensity data D21, and the light intensity data D22

↓

Obtaining hematocrit from a relationship curve according to the absorbance $A$

FIG. 1

FIG. 2

FIG. 3

## INTERNATIONAL SEARCH REPORT

| International application No. |
| --- |
| **PCT/CN2023/105029** |

**A. CLASSIFICATION OF SUBJECT MATTER**

G01N 21/31(2006.01)i

According to International Patent Classification (IPC) or to both national classification and IPC

**B. FIELDS SEARCHED**

Minimum documentation searched (classification system followed by classification symbols)

IPC,CPC: G01N

Documentation searched other than minimum documentation to the extent that such documents are included in the fields searched

Electronic data base consulted during the international search (name of data base and, where practicable, search terms used)

CNTXT, ENTXTC, ENTXT, VEN, CNKI: 暗电流, 暗流, 百分比, 比容, 不同, 穿透, 第二, 方位, 方向, 红细胞, 角度, 全血, 透射, 吸, 吸附作用, 吸光, 吸光度, 吸收, 吸收度, 吸收率, 血, 压积, 占比, HCT, hematocrit, dark, current, flow, percentage, volume, different, penetrat+, second, orientat+, direction, blood, cell, angle, transmission, absorption, absorbance, fraction

**C. DOCUMENTS CONSIDERED TO BE RELEVANT**

| Category* | Citation of document, with indication, where appropriate, of the relevant passages | Relevant to claim No. |
| --- | --- | --- |
| PX | CN 115452744 A (SHENZHEN DRAWRAY BIOTECH CO., LTD.) 09 December 2022 (2022-12-09)<br>    description, paragraphs [0039]-[0099], and figures 1-2 | 1-11 |
| X | CN 113533226 A (SHENZHEN MINDRAY BIO-MEDICAL ELECTRONICS CO., LTD. et al.) 22 October 2021 (2021-10-22)<br>    description, paragraphs [0098]-[0099] and [0162]-[0232], and figure 9 | 8-11 |
| A | CN 108738338 A (SIEMENS HEALTHCARE DIAGNOSTICS INC.) 02 November 2018 (2018-11-02)<br>    entire document | 1-11 |
| A | CN 113533244 A (SHENZHEN INSTITUTE OF ADVANCED TECHNOLOGY, CHINESE ACADEMY OF SCIENCES) 22 October 2021 (2021-10-22)<br>    entire document | 1-11 |
| A | CN 114034653 A (TSINGHUA UNIVERSITY) 11 February 2022 (2022-02-11)<br>    entire document | 1-11 |

☑ Further documents are listed in the continuation of Box C.　　☑ See patent family annex.

| | |
| --- | --- |
| *　Special categories of cited documents:<br>"A"　document defining the general state of the art which is not considered to be of particular relevance<br>"D"　document cited by the applicant in the international application<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search | Date of mailing of the international search report |
| --- | --- |
| **10 November 2023** | **14 November 2023** |

| Name and mailing address of the ISA/CN | Authorized officer |
| --- | --- |
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/105029**

**C.** **DOCUMENTS CONSIDERED TO BE RELEVANT**

| Category* | Citation of document, with indication, where appropriate, of the relevant passages | Relevant to claim No. |
|---|---|---|
| A | US 2016238521 A1 (INSTITUTE NATIONAL DE LA SANTE ET DE LA RESEARCH MEDICAL et al.) 18 August 2016 (2016-08-18)<br>entire document | 1-11 |

Form PCT/ISA/210 (second sheet) (July 2022)

INTERNATIONAL SEARCH REPORT

Information on patent family members

International application No.

**PCT/CN2023/105029**

| Patent document cited in search report | | | Publication date (day/month/year) | Patent family member(s) | | | Publication date (day/month/year) |
|---|---|---|---|---|---|---|---|
| CN | 115452744 | A | 09 December 2022 | None | | | |
| CN | 113533226 | A | 22 October 2021 | None | | | |
| CN | 108738338 | A | 02 November 2018 | EP | 3408651 | A1 | 05 December 2018 |
| | | | | EP | 3408651 | A4 | 12 December 2018 |
| | | | | HK | 1257096 | A1 | 11 October 2019 |
| | | | | US | 2018372715 | A1 | 27 December 2018 |
| | | | | US | 10816538 | B2 | 27 October 2020 |
| | | | | WO | 2017132169 | A1 | 03 August 2017 |
| | | | | JP | 2019511700 | A | 25 April 2019 |
| | | | | JP | 6791972 | B2 | 25 November 2020 |
| CN | 113533244 | A | 22 October 2021 | None | | | |
| CN | 114034653 | A | 11 February 2022 | None | | | |
| US | 2016238521 | A1 | 18 August 2016 | EP | 3058368 | A1 | 24 August 2016 |
| | | | | WO | 2015056190 | A1 | 23 April 2015 |
| | | | | US | 9909977 | B2 | 06 March 2018 |

Form PCT/ISA/210 (patent family annex) (July 2022)

**EP 4 534 979 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022111226129